(19) Europäisches Patentamt / European Patent Office / Office européen des brevets

(11) **EP 3 329 306 B1**

(12) **EUROPEAN PATENT SPECIFICATION**

(45) Date of publication and mention
of the grant of the patent:
**13.10.2021  Bulletin 2021/41**

(51) Int Cl.:
**G01V 9/00** *(2006.01)*          **B01D 53/62** *(2006.01)*
**E21B 41/00** *(2006.01)*

(21) Application number: **16832007.5**

(22) Date of filing: **28.07.2016**

(86) International application number:
**PCT/CA2016/050883**

(87) International publication number:
**WO 2017/020120 (09.02.2017 Gazette 2017/06)**

(54) **METHOD FOR CALCULATING NET CARBON CAPTURE FROM OCEAN IRON FERTILIZATION**

ERFAHREN ZUR BERECHNUNG DER KOHLENSTOFFNETTOABSCHEIDUNG DURCH
OZEANEISENDÜNGUNG

PROCÉDÉ POUR CALCULER LE CARBONE CAPTURÉ NET PAR LA FERTILISATION EN FER DE
L'OCÉAN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **31.07.2015  CA 2899051**

(43) Date of publication of application:
**06.06.2018  Bulletin 2018/23**

(73) Proprietor: **Lucent Biosciences, Inc.**
**West Vancouver, British Columbia V7T 1B9 (CA)**

(72) Inventor: **GROSS, Peter**
**West-Vancouver, British Columbia V7T 1B9 (CA)**

(74) Representative: **Manuel Illescas y Asociados, S.L.**
**C/ Príncipe de Vergara 197, Oficina 1°A**
**28002 Madrid (ES)**

(56) References cited:
**WO-A1-2008/124883      WO-A2-2008/131455**
**CA-A1- 2 835 792**

- **VICTOR SMETACEK ET AL: "Deep carbon export
  from a Southern Ocean iron-fertilized diatom
  bloom", NATURE, vol. 487, no. 7407, 1 January
  2012 (2012-01-01), pages 313-319, XP055361696,
  London ISSN: 0028-0836, DOI:
  10.1038/nature11229**
- **SMETACEK ET AL.: 'Deep carbon export from a
  Southern Ocean iron-fertilized diatom bloom'
  NATURE vol. 487, 19 July 2012, pages 313 - 319,
  XP055361696**

**Description**

**FIELD OF THE INVENTION**

This invention, related to greenhouse gas reduction, carbon offset methodology, carbon sequestration, environmental science and environmental sustainability, is in the field of enhancement of atmospheric carbon sequestration by enhanced photosynthetic productivity through the maximized iron fertilization of oceanic waters.

**BACKGROUND OF THE INVENTION**

Carbon sequestered through oceanic processes may also be known as "Blue Carbon". One reason to engage in Ocean Iron Fertilization (OIF) is to facilitate the drawdown of atmospheric $CO_2$ in nutrient-rich, low chlorophyll (HNLC[1]) regions of the ocean.
[1] Smetacek, V. et al. Deep carbon export from a Southern Ocean iron- fertilized diatom bloom. Nature, 2012, 487: p. 313-319.
There are two aspects of atmospheric carbon dioxide removal:
The first component is carbon dioxide uptake and conversion to organic carbon in response to photosynthetic activity (phytoplankton bloom) in euphotic waters. This results in a drawdown of the $CO_2$ partial pressure in the surface ocean, the generation of a negative gradient across the air-sea interface, and a net flux (uptake) of $CO_2$ from the atmosphere.
The second component is the transfer of a portion of the phytoplankton organic carbon to the deep ocean (carbon export) below the permanent thermocline where it will be sequestered and isolated from the atmosphere for a period of time measured in centuries to millennia depending on ocean circulation patterns in the project location.
The combination of these two components is often referred to as the $CO_2$ biological pump and is one of the processes by which the oceans take up some of the anthropogenic $CO_2$ emitted to the atmosphere. In fact, it is estimated that the oceans have absorbed approximately 30% of the anthropogenic $CO_2$ released to the atmosphere since the beginning of the industrial revolution (Sabine et al. 2004)[2].
[2] Sabine, C.L., et al., The oceanic sink for anthropogenic CO2. Science, 2004. 305: p. 367-371.
12 IOF[3] experiments that have been carried out to date have led to increased photosynthetic activity (i.e., increased chlorophyll concentrations). Three of these thirteen studies [Boyd et al., 2004[4]; Bishop et al., 2004[5];Smetacek et al., 2012[6]] reported net, but varying carbon export [Williamson et al., 2012][7].
[3] Ironex I, Ironex II, SOIREE (Southern Ocean Iron Release Experiment), EisenEx, SEEDS (Subarctic Pacific Iron Experiment for Ecosystem Dynamics Study), SOFeX (Southern Ocean Iron Experiments - North and South), SERIES (Subarctic Ecosystem Response to Iron Enrichment Study), SEEDS-II, EIFEX (European iron Fertilization Experiment), CROZEX (CROZet natural iron bloom and Export experiment), LOHAFEX, HSRC (Haida Salmon Restoration Coroporation)
[4] Boyd, P.W., et al., The decline and fate of an iron-induced subarctic phytoplankton bloom. Nature, 2004. 428: p. 549-553.
[5] Bishop, J., et al., Robotic observations of enhanced carbon biomass and export at 55°S during SOFeX. Science, 2004. 304: p. 417-420.
[6] Smetacek, V. et al. Deep carbon export from a Southern Ocean iron- fertilized diatom bloom. Nature, 2012, 487: p. 313-319.
[7] Williamson, P., et al., Ocean Fertilization for geoengineering: A review of effectiveness, environmental impacts and emerging governance., Process Safety and Environmental Protection, 2012m 475-488.
Nonetheless, is some cases, up to 50% of the bloom biomass generated by IOF sank below 1000m depth [Smetacek et al. 2012, Smetacek et al. Submitted][4] [8], and similar longer term observations have been reported following natural blooms [Blain et al., 2007][9].
**[8] Smetacek, V., et al., Massive carbon flux to the deep sea from an iron- fertilized** phytoplankton bloom in the Southern Ocean. (submitted)
[9] Blain, S., et al., Effect of natural iron fertilization on carbon sequestration in the Southern Ocean. Nature, 2007. 446(26 April): p. 1070-1074.
Recent measurements of carbon export from naturally occurring seasonal phytoplankton blooms in the northwest Pacific and subtropical Pacific suggest that the biological pump is much more efficient than previously thought. In an experiment named VERTIGO (Vertical Transport in the Global Ocean), novel techniques, including neutrally buoyant sediment traps, were used to look at the fate of organic carbon below the mixed layer, and found that export to the deep ocean (below 500 m) was 2-5 times greater than previously thought [Buesseler et al., 2007][10]
[10] Buesseler, K.O., et al., Revisiting carbon flux through the Ocean's twilight zone. Science, 2007.316(5824): p. 567-570.
Similarly, observations of blooms stimulated by iron fertilization in the Southern Ocean (e.g. over the Kerguelen plateau) showed extremely high rates of carbon export compared to prior observations [Blain et al., 2007][11].
[11] Blain, S., et al., Effect of natural iron fertilization on carbon sequestration in the Southern Ocean. Nature, 2007. 446(26 April): p. 1070-1074.

Recently, numerical models have been used to simulate the ecological response to the natural iron cycle. When coupled to ocean circulation and biogeochemical models, these simulations provide more realistic predictions of surface ocean $CO_2$ drawdown than previously possible [Jin et al., 2008[12]; Aumont and Bopp, 2006[13]; Zahariev et al., 2008[14]]. The results of the Aumont and Bopp [2006] simulations suggest that a full ocean OIF during 100 years would remove 33 ppm of $CO_2$ from the atmosphere. This corresponds to slightly more than 25% of the increase in atmospheric $CO_2$ levels since the early 19th century. Zahariev et al. [2008], using a different set of model assumptions, calculated that global OIF would enhance $CO_2$ uptake by approximately 11% of the 2004 annual anthropogenic emissions, but could only be sustained at that level for a year or two under continuous fertilization [Zahariev, Christian, and Denman, 2008].

[12] Jin, X., et al., The impact on atmospheric CO2 of iron fertilization induced changes in the ocean's biological pump. Biogeosciences, 2008. 5: p. 385-406.

[13] Aumont, O. and L. Bopp, Globalizing results from ocean in situ iron fertilization studies. Global Biogeochem. Cycles, 2006. 20 (GB2017).

[14] Zahariev, K., J.R. Christian, and K.L. Denman, Preindustrial, historical, and fertilization simulations using a global ocean carbon model with new parameterizations of iron limitation, calcification, and N2 fixation. Progress in Oceanography, 2008. 77: p. 56-82.

Although much smaller, this quantity is still equivalent to that of many other emission reduction strategies, such as moderate and low penetration wind power. Therefore, classic OIF is one, but not the best, mitigation technique that could be applied to reduce global atmospheric $CO_2$ levels.

PERMANENCE

There are two components to the question of 'permanence' of carbon sequestration from OIF. The first component is the length of time that sequestered carbon will be prevented from returning to the atmosphere. Sequestration time is a function of particulate organic carbon settling depth, itself a function of the settling rate (taxonomy, grazing, particle density, aggregation, ballasting) and the ocean circulation patterns below the fertilized patch. Deep ocean mixing is a slow process that occurs on a time scale of hundreds to a thousand years.

The ability to associate the depth of the water column with age (of last contact with the atmosphere) and future trajectory of the water is well established in the oceanographic community. Measurements of the intrusion or evasion rate of natural (e.g., radon, 14C) and man made tracers, e.g., tritium, CFCs (chlorofluorocarbon), SF6 (sulphur hexasulfide) into the world oceans provide calibration data for circulation models. These models can then produce a "residence time vs. depth profile" curve for any area of the ocean in which ocean fertilization is conducted [England, 1995; Matsumoto, 2007; Fine, 2011; Khatiwala et al., 2012], as well as the general future path of this parcel of water.

The residence time of carbon sequestered from OIF is calculated through application of general ocean circulation models such as those described above.

As organic carbon particles, produced in the euphotic zone, sink through the water column, they are subject to microbial respiration or remineralization as the organic material is converted back to its inorganic constituents, including $CO_2$.

Most of the sequestered carbon is remineralized close to the ocean surface (top few hundred meters), but a significant fraction (~1-10%) can sink to the deep ocean or even the sea floor. The period of carbon sequestration from OIF is defined by the future trajectory of the parcel of water in which each unit of organic carbon is remineralized.

In order to generate carbon credits from OIF, carbon sequestration should be measured at depth corresponding to a desired residence time period. The second component of 'permanence' of carbon sequestration is the definition of a permanence time period. The Kyoto Protocol uses 100 years as the arbitrary time horizon for which the Global Warming Potential (GWP) of the six regulated GHGs are normalized [UNFCCC, 1997]. This choice by the Kyoto Protocol policy makers incorporated consideration for both long term benefits and short term benefits of climate mitigation options [IPCC, 1995; p.229], and is the best definition available for viable carbon sequestration.

To be consistent with global carbon policy, a depth corresponding to a 100 year residence time should be considered on a case by case basis. [15] [16] [17] [18] [19] [20]

[15] England, M.H., The Age of Water and Ventilation Timescales in a Global Ocean Model. Journal of Physical Oceanography, 1995. 25(November): p. 2756 - 2777.

[16] IPCC, Climate Change 1994: Radiative Forcing of Climate Change and an Evaluation of the IPCC IS92 Emission Scenarios, ed. J.T. Houghton. 1995, Cambridge, U.K.: Cambridge University Press.

[17] Matsumoto, K., Radiocarbon based circulation age of the world oceans. Journal of Geophysical Research - Oceans, 2007. 112(C09004).

[18] UNFCCC, REPORT OF THE CONFERENCE OF THE PARTIES ON ITS THIRD SESSION, HELD AT KYOTO FROM 1 TO 11 DECEMBER 1997, in FCCC/CP/1997/7/Add.1, UNFCCC, Editor. 1997

[19] Fine R., Observations of CFCs and SF6 as Ocean Tracers. Annual Review in Marine Science, 2011. 3: p. 173-195

[20] Khatiwala, S., F. primeau and M. Holzer., Ventilation of the deep ocean constrained with tracer observations and implications for radiocarbon estimates of ideal mean age. Earth and Planetary Science Letters, 2012. 325-326: p. 116-125.

BRIEF DESCRIPTION OF THE DRAWINGS

Figure 1: A) Example of an ocean eddy. B) The image shows surface sea height (SSH) as measured from satellite observations; the arrow and circled area highlights an ocean eddy that is proud of the sea surface by approximately 20 cm.

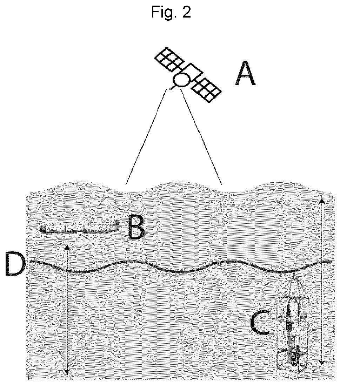

Figure 2: Shows a diagram of how various oceanographic parameters shall be measured. **"A"** shows satellite observations being taken of the project area. These observations shall include Chlorophyll (chl), photosynthetically active radiation (par) , surface sea temperature (sst) and sea surface height (ssh). In-situ instruments such as **"B"** autonomous underwater vehicles that are able to move vertically in the water column or **"C"** vertically moving instruments lowered from a surface vessel may be used to obtain data to substitute for satellite observations should satellite observations not be available of the project area. Observations of organic carbon near the surface and in proximity to the thermocline **"D"** shall be taken to determine the vertical carbon flux through the water column. Vertically moving instruments such as **"C"** or **"B"** when suitably equipped may be used to obtain vertical carbon flux measurements by taking measurements of particulate organic carbon (POC) and dissolved organic carbon (DOC).

Figure 3: Ocean eddy and project boundary defined at the Initial stage of the project (initial project boundary).

Figure 4: Project Boundary as net primary production increases. The project area spread beyond the initial project boundary. Ocean eddy remains the same.

Figure 5: Increases over time of the area contained within the Actual Project Boundary, the Net Primary Production (NPP ) will cease to be 10% greater that surrounding waters. Ocean eddy remains the same.

The present invention provides a method for calculating the quantity of atmospheric carbon sequestration based on the measurements obtained that allows determining the net quantity of atmospheric carbon that is sequestered.

DETAILED DESCRIPTION OF THE INVENTION

While certain preferred embodiments of the present invention may be described, modifications, adaptations, and other embodiments are also possible. For example, the methods and processes described herein may be modified by substituting, reordering, or adding stages to the disclosed methods.
Therefore, the following detailed description does not limit the scope of the invention. In the light of the present description, the following terms or phrases should be understood with the meanings described below:
The term "Ocean Eddy" as used herein means an ocean surface sea height anomaly. An ocean eddy is and is a circular current of water that cause nutrients that are normally found in colder, deeper waters to come to the surface of the ocean, the water within an eddy usually has different temperature and composition characteristics to the water outside of the eddy.
The term "CTD package" as used herein corresponds to a large instrument package for acquiring water column profiles, as understood according to the standard definition used by oceanographers, where CTD corresponds to the minimum measures: electrical conductivity, temperature, and depth (pressure).

Project boundary

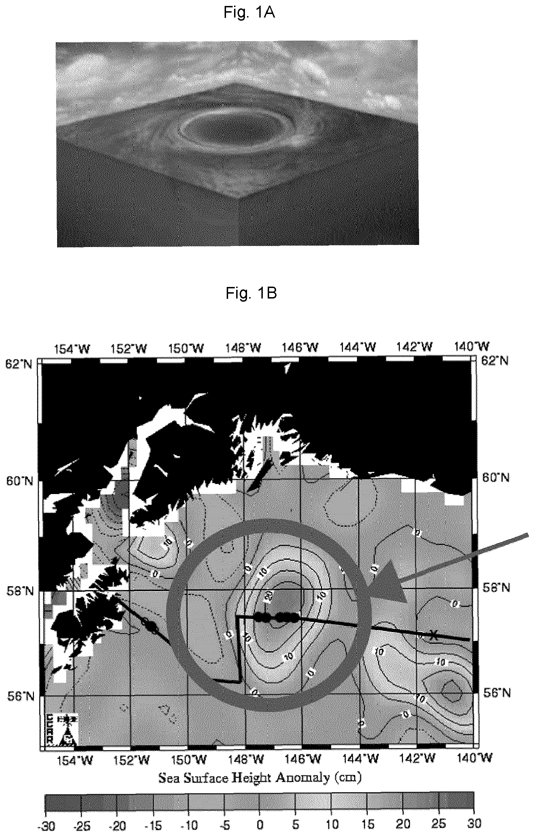

The project boundary according to the present invention is unique from most other carbon methodologies. Because oceanic carbon sequestration uses areas of the ocean as a project location, the circulation of the ocean water and currents will mean the project boundary will move. However, it is still possible to define a project boundary explicitly.
Iron enrichment of the ocean shall take place in an ocean eddy. Ocean eddies are circular currents of water that are not flush with the ocean surface (see Figures 1A and 1B). Although difficult to define with the naked eye, ocean eddies may be defined using Surface Sea Height (ssh) data available from public domain sources[21].
A surface sea height anomaly of 3cm or greater shall be considered to be the criteria for defining the border of the ocean eddy. The area enclosed within this border shall be defined as the initial project location.
Ocean eddies have desirable properties when engaging in Iron enrichment. They resist mixing of the nutrients and Iron into the surrounding waters, and are sources of upwelling nutrients that stimulate photosynthesis.[4]

For these reasons, the project shall be conducted within an ocean eddy.

An iron compound is introduced into the initial project boundary in order to stimulate photosynthetic activity within this boundary. Addition of Iron into an eddy will stimulate photosynthesis and shall create an increase in Net Primary Production (NPP), however this effect is not permanent. The project boundary will therefore cease to exist once the effect of Iron enrichment is no longer significantly elevated from surrounding waters.

NPP shall be calculated prior to the introduction of iron compound. This shall be considered the background or pre-existing NPP of the initial project boundary. As the iron compound increases NPP, the area of improved photosynthesis will spread beyond the initial project boundary.

Repeated observations of sea surface chlorophyll within and surrounding the initial project boundary shall be performed to determine the delineation between the area of increased photosynthesis and pre-existing photosynthesis. This delineation shall be defined as the actual project boundary. The criteria used to determine the delineation of the actual project boundary shall be an increase of NPP that is 10% or greater than the background NPP, or the NPP surrounding the actual project boundary. The actual

[21] http://oceanmotion.org/html/resources/ssedv.htm

project boundary therefore shall be the area enclosed by this delineation, and will change over time.

When the NPP within the actual project boundary is no longer elevated more than 10% of the NPP surrounding the actual project boundary, the project shall be considered concluded.

Observations for calculating NPP within the actual project boundary and observations for calculating NPP surrounding the actual project boundary shall be taken daily or interpolated daily to create a time-series that defines the area of the actual project boundary on a daily basis from the introduction of iron compound until project conclusion. Because observations may not be possible to accomplish on a daily basis due to environmental conditions and measurement device limitations, it shall be acceptable to use interpolation and extrapolation from known data points to estimate daily NPP metrics.

Therefore, the project boundary shall be defined in the following steps;

a) A high nutrient, low chlorophyll (HNLC) ocean eddy in pelagic waters shall be selected as a project location.

b) A baseline of Net Primary Production (NPP) within the ocean eddy shall be obtained prior to Iron enrichment (NPP is explained later in this document).

c) As Iron enrichment is performed, the Net Primary Production (NPP) will increase within the eddy to a maximum value ($NPP_{max}$) after which it will decrease until it is indistinguishable from adjacent waters.

d) The project boundary therefore shall be defined as a delineation around the Iron enriched ocean eddy, where the NPP is 10% or greater than surrounding waters.

e) Once the NPP within the ocean eddy has reduced to less than 10% of surrounding waters, the project shall be considered to have reached it's conclusion.

Baseline scenario

The carbon export from an Ocean Iron enrichment project is the mass balance obtained from difference between the initial Net Primary Production (NPP) within the project boundary, the adjacent NPP once the project has commenced and the increased NPP within the project boundary caused by Iron enrichment.

Therefore, prior to commencement of the project, measurements and metrics of the project area shall be undertaken to allow the NPP of the project area to be established. During the project, measurements and metrics of the adjacent ocean waters shall be undertaken to account for changing NPP that could have been reasonably expected prior to execution of the project. This shall be defined as the baseline NPP.

**Metrics to be measured:**

| Data / parameter: | chl |
| --- | --- |
| Data unit: | $mg/m^3$ |
| Description: | Chlorophyll a Concentration |
| Source of data: | Satellite observations of the sea surface, or direct in-situ measurements from Chlorophyll a measurement devices deployed on surface ships or other means by project participants. |
| Measurement procedures (if any): | If environmental factors such as overcast or storms prevent occasional observations from being made, extrapolation from known data points may be substituted. |

(continued)

| Data / parameter: | chl |
|---|---|
| Monitoring frequency: | Daily if possible. |
| QA/QC procedures: | Pre or post calibration of measurement instruments must be made within 60 days when in-situ instruments are used. |

| Data / parameter: | par |
|---|---|
| Data unit: | mol quanta/m$^2$ (Einsteins per day per square meter) |
| Description: | Photosynthetically Active Radiation |
| Source of data: | Satellite observations of the sea surface, or direct in-situ measurements from PAR measurement devices deployed on surface ships or other means by project participants. |
| Measurement procedures (if any): | If environmental factors such as overcast or storms prevent occasional observations from being made, extrapolation from known data points may be substituted. |
| Monitoring frequency: | Daily if possible. |
| QA/QC procedures: | Pre or post calibration of measurement instruments must be made within 60 days when in-situ instruments are used. |

| Data / parameter: | sst |
|---|---|
| Data unit: | Degrees centigrade |
| Description: | Sea Surface Temperature |
| Source of data: | Satellite observations of the sea surface temperature, or direct in-situ measurements from temperature measurement devices deployed on surface ships or other means by project participants. |
| Measurement procedures (if any): | If environmental factors such as overcast or storms prevent occasional observations from being made, extrapolation from known data points may be substituted. |
| Monitoring frequency: | Daily if possible. |
| QA/QC procedures: | Pre or post calibration of measurement instruments must be made within 60 days when in-situ instruments are used. |

| Data / parameter: | POC |
|---|---|
| Data unit: | mg/m$^3$ |
| Description: | Particulate Organic Carbon vertical flux |
| Source of data: | Direct in-situ measurements from nets, water samples or measurement devices deployed on surface ships or other means by project participants. |
| Measurement procedures (if anv): | If environmental factors such as overcast or storms prevent occasional observations from being made, extrapolation from known data points may be substituted. |
| Monitoring frequency: | As frequently as possible. |

| Data / parameter: | DOC |
|---|---|
| Data unit: | mg/m$^3$ |

(continued)

| Data / parameter: | DOC |
|---|---|
| Description: | Dissovled Organic Carbon vertical flux |
| Source of data: | Direct in-situ measurements from nets, water samples or measurement devices deployed on surface ships or other means by project participants. |
| Measurement procedures (if anv): | If environmental factors such as overcast or storms prevent occasional observations from being made, extrapolation from known data points may be substituted. |
| Monitoring frequency: | As frequently as possible. |

| Data / parameter: | Day length or Photoperiod |
|---|---|
| Data unit: | Decimal hours, hours of sunlight per day |
| Description: | The interval in a 24 hour period during which phytoplankton is exposed to light |
| Source of data: | A widely available environmental metric |
| Measurement procedures (if anv): | - |
| Monitoring frequency: | Daily |

The method and calculations for establishing NPP are defined later.

Additionalitv

Because execution of a pelagic ocean Blue Carbon project is at sea, it requires substantial resources, and cannot be performed 'accidentally' or through means other than specifically engaging in the project, therefore, the additionality is satisfied.

Project emissions

Project emissions are the carbon emissions that will be emitted through the course of execution of the project. The largest source of carbon emissions is expected to be exhaust from internal combustion engines used to power seagoing vessels used in the project. This quantity will vary depending on the type of vessel used, and the duration of the seagoing voyages, and will be calculated on a case-by-case basis. The project emissions are estimated to be a minor fraction of the project sequestration.

Leakage

The primary source of leakage is remineralization of vertical carbon transport back into the ecosystem. Leakage is removed from the project carbon calculations through the term "Export Efficiency" which defines the actual vertical carbon transport that is sequestered, which accounts for remineralization.

Method for calculating the quantity of atmospheric carbon sequestration

In order to calculate the carbon that is removed from the atmosphere, it is necessary to obtain measurements of various oceanographic parameters, and apply those measurements to a mathematical calculation that provides an estimate of the carbon sequestered.

However, due to the difficulty to obtain certain metrics and measurements from the open ocean, the calculation described in this methodology must adhere to two principles for practical application. These are;

(1) The metrics used to perform the calculation described in this methodology must be observable. That means that there must be a practical method to gather the data destined as inputs to the calculation and they must be easy to obtain with currently available technology. Therefore, the calculation provides an estimate of carbon fixation that is a 'best possible' approximation.

(2) Secondly, the calculation must be based upon a scientifically accepted algorithm that provides realistic estimates of photosynthetic production. This calculation uses estimates of Net Primary Production (NPP), to evaluate the quantity of carbon fixed by ocean photosynthesis per unit area and time.

The main metrics that have to be acquired correspond to:

- Chlorophyll concentration (*chl*)
- Photosynthetic active radiation (*par*)
- Surface sea temperature (*sst*)
- Day length
- Particulate Organic Carbon (POC)
- Dissolved Organic Carbon (DOC)
- Organic carbon in the Euphotic zone (CorgE)
- Organic carbon in the close to the deep Thermocline (CorgT)

These observations shall be used as input data into a calculation to determine Net Primary Production (NPP) and carbon transport efficiency. These measurements shall have a minimum geographical resolution of 10 square kilometers per observation or better.

The observations should be repeated daily or interpolated daily using a suite of instruments and sampling mechanisms.

These observations shall be made from public or private data sources. In the absence of public or private data observations, an acceptable substitute may be obtained from

Autonomous Underwater Vehicles (AUV's) equipped with instrumentation capable of measuring the parameters.

The use of AUV data collection conducted concurrently with satellite observations will provide greater resolution of sea metrics.

Alternatively, sediment traps, buoys, shipboard instruments, niskin bottles and/or a surface vessel may be used, which has been equipped with instrumentation able to measure these parameters.

A fraction of the carbon fixed in the euphotic zone will sink and pass through the deep thermocline where it may be sequestered for a significant amount of time (months or years). Although there are several different methods to perform this calculation, the methodology described in the present invention will use the Eppley VGPM Net Primary Production (NPP) calculation[22], which is widely used and peer reviewed[23].

[22] Eppley, RW. Temperature and phytoplankton growth in the sea. Fishery Bulletin, 1972, Volume 70: 1063-1085

[23] Behrenfeld, MJ., PG Falkowski., Photosynthetic rates derived from satellite-based chlorophyll concentration. Limnology and Oceanography, 1997a, Volume 42: 1-20

NPP calculation explanation

The Eppley Net Primary Production (NPP) calculation utilizes the Vertically Generalized Production Model (VGPM) proposed by Behrenfeld and Falkowski in 1997[14], and is commonly used to estimate regional to global ocean NPP. This algorithm is based primarily on observations of chlorophyll concentrations, which may be obtained from satellite, aircraft or in situ surveys, as shown in Figure 2. The foundation of the VGPM model is that NPP varies in a predictable manner with chlorophyll concentration (*chl*), i.e., NPP is a function dependent from the chlorophyll concentration.

$$\textbf{NPP} = \textbf{f} \, (\textbf{\textit{chl}})$$

The *Eppley* VGPM model is a version of the Behrenfeld and Falkowski model that includes the temperature dependent growth function of phytoplankton described by Eppley (1972)[13]. Thus, the effects of ocean temperature on carbon fixation are taken into account.

The algorithm employs a variable termed pb_opt, which is the maximum daily net primary production found within a given water column, and is based on the curvature of the temperature dependent phytoplankton growth function described by Eppley (1972). It is typically expressed in units of mg carbon fixed per mg of chlorophyll per hour.

NPP per volume unit at the depth of pb_opt is thus:

$$\textbf{NPP} = \textbf{\textit{chl}} * \textbf{pb\_opt} * \textbf{day length}$$

Day length is the number of hours of daylight at the location of interest and NPP is the number of milligrams of carbon fixed per day per unit volume. In order to relate the total water column volume to surface area, a *water column integrated productivity per unit of ocean area* function is required. This essentially projects the volume of the area of interest to a value expressed as production per unit surface area.

$$\textbf{NPP} = \textit{chl} * \textbf{pb\_opt} * \textbf{day length} * \textbf{volume function}$$

The volume function may be explained as follows. Photosynthesis through the water column is not uniform. This is because photosynthetic activity is driven by sunlight. As sunlight penetrates the water column, some of it will be absorbed and some is scattered backwards. Consequently, sunlight decreases rapidly with depth in a near exponential manner. Furthermore, sunlight intensity varies due to effects such as cloud cover. These effects of light on photosynthesis are accounted for in the VGPM algorithm by including a light dependent term, f(*par*), also known as the photosynthetic active radiation, in the calculation.

$$\textbf{Volume function} = \textbf{f}(\textit{par}) * \textbf{z\_eu}$$

where z_eu is the *euphotic depth* at which 1% of surface/incident light is available. The z_eu term is calculated using the Morel and Berthon (1989) case 1 model[24]. This model estimates z_eu from surface chlorophyll concentrations and is based on empirical equations fitted to observational data. This term distinguishes between lower and higher chlorophyll waters. Given the amount of chlorophyll, the euphotic depth is estimated using distinct equations for lower and higher chlorophyll.

If *chl* < 1

$$\textbf{chl\_tot} = \textbf{38} * \textit{chl}^{0.425}$$

if *chl* >= 1 chl_tot = 40.2 * *chl*$^{0.507}$

$$\textbf{z\_eu} = \textbf{200} * \textbf{(chl\_tot)}^{-0.293}$$

if z_eu <= 102,
then,

$$\textbf{z\_eu} = \textbf{568.2} * \textbf{(chl\_tot)}^{-0.746}$$

The f(*par*) term is the ratio of water column integrated NPP to the maximum potential NPP if photosynthetic rates were maintained at maximum levels (i.e. pb_opt) throughout the water column. This lightdependent term was determined empirically using thousands of field productivity measurements and is given by:

$$f(par) = 0.66125 * \frac{par}{(par + 4.1)}$$

Replacing the "volume function" with these relationships yields the Eppley VGPM relationship:

[24] Morel, A., J-F Berthon., Surface pigments, algal biomass profiles, and potential production of the euphotic layer: Relationships reinvestigated in view of remote-sensing applications. Limnology and Oceanography. 1989, Volume 34: 1545-1562

$$NPP = chl * pb\_opt * day\ length * \left[0.66125 * \frac{par}{(par + 4.1)}\right] * z\_eu$$

The pb_opt term, as defined by Eppley(1972), expresses photosynthetic activity as a function of sea surface temperature (*sst*) and is given by:

$$pb\_opt = 1.54 * 10^{|(0.0275 * sst) - 0.07|}$$

Therefore, the NPP calculation requires the following input data fields:

- **chl**: chlorophyll concentration at the surface of the ocean (measured from satellite observations, aerial instrumentation or in situ instruments)
- **par:** photosynthetically active radiation (measured from satellite observations, aircraft, aerial instrumentation or in situ instruments)
- **sst:** surface sea temperature (measured from satellite observations, aerial instrumentation or in situ instruments)
- **day length,** (widely published and commonly available environmental metric)

Carbon sequestration

NPP provides the quantity of carbon fixed per unit area, but this is not the same as carbon sequestration. Not all of the carbon manifested by NPP will sink through the water column and reach the deep thermocline. In addition, the ratio of carbon sequestered to NPP may vary depending on environmental conditions and a fixed *carbon sequestration: NPP* ratio may not apply in all conditions and locations. Therefore, a conversion ratio that relates carbon sequestration to NPP for the area under study must be established.

The vertical carbon flux through the water column may be estimated from Particulate Organic Carbon (POC) and Dissolved Organic Carbon (DOC) measurements taken at various depths within the water column. Preserving principle (1), that metrics used in the calculation must be observable, commonly available in situ oceanographic instruments (such as gliders, water samples, sediment traps or CTD packages) will be utilized to measure POC and DOC at various depths, covering the euphotic zone to the depth of the permanent thermocline.

Sub-surface particulate organic carbon (POC) and dissolved organic carbon (DOC) observations shall be taken from measurements in the euphotic zone of the sub-surface, and also in proximity of the deep thermocline of the sub-surface.

Observations shall be taken within the actual project boundary and surrounding the actual project boundary to determine the vertical carbon flux within the project boundary and surrounding the project boundary.

Measurements of POC and DOC concentrations at various depths in the water column will yield estimates of the organic carbon flux to the deep sea and the efficiency of carbon sequestration. Organic Carbon (c_org) is expressed as:

$$\mathbf{c\_org = POC + DOC}$$

The maximum vertical carbon flux through the water column is the c_org present in the euphotic zone in the area under study. This metric may be determined by in situ measurements in the euphotic zone. This term shall be CorgE.

The actual carbon flux at the deep thermocline may be determined by in situ measurements of Corg in proximity to the deep thermocline. This term shall be CorgT.

During the measurement process, sufficient time (weeks to months) must be allowed for carbon flux to move vertically from the euphotic zone to the deep thermocline. A ratio may now be calculated that defines the carbon vertical transport efficiency in the area under study. This term shall be CorgEff.

$$\mathbf{CorgEff = CorgT/CorgE}$$

Therefore, *total carbon sequestration* (Cseq) is therefore defined as:

$$\mathbf{Cseq = CorgEff * NPP}$$

Preserving principle 1, CorgEff will be considered a constant within the area under study. Therefore, once this metric has been established, NPP will be calculated using the most granular satellite or other remote sensing observations available, but preserving CorgEff as a locally defined constant. This metric is applicable only for the time and place under observation and must be supported by in situ measurements (gliders, water samples, CTD packages, sediment traps, etc.)

However, the total project carbon sequestration (Cproj) must remove the baseline carbon export that the project area would sequester without the influence of Iron enrichment.

Therefore;

$$Cproj = Cseq - Cbaseline$$

Where, Cbaseline is calculated in the same manner as Cseq, but using metrics and observations of the project area prior to Iron enrichment.

The carbon transport efficiency within the project boundary shall be defined as CorgEff(P) and the carbon transport efficiency outside the project boundary shall be used as a baseline metric and shall be defined as CorgEff(B).

Total daily carbon sequestration within the actual project boundary shall be the total NPP, discounted by the vertical carbon transport efficiency within the actual project boundary. Therefore, the total daily carbon sequestration within the actual project boundary shall be:

$$Cseq(P) = CorgEff(P) * NPP$$

Similarly, total daily carbon sequestration within the actual project boundary shall be:

$$Cseq(B) = CorgEff(B) * NPP$$

Thus, the net daily project carbon sequestration shall be the daily carbon sequestration inside the actual project boundary, minus the daily baseline carbon sequestration outside the actual project boundary. Therefore, the total net carbon sequestration shall be defined as:

$$Cseq(NET) = Cseq(P)-Cseq(B).$$

Cseq(NET) shall be calculated daily from the date of introduction of iron compound, until the date of project conclusion. The project duration (Days(P)) shall be defined as the number of days from introduction of iron compound until project conclusion.

The total net carbon sequestration of the project (Ctotal) shall be the daily Cseq(NET) summed daily, from the introduction of iron compound until project conclusion.

Thus, the method for determining net quantity of sequestered atmospheric carbon can be described according to the following steps:

a) defining a project boundary by

- obtaining Satellite surface sea height (SSH) observations from public or private data sources to determine the location of an Ocean Eddy that may be used as a project location;
- selecting an Ocean Eddy existing within a region of the pelagic ocean considered to be High Nutrient Low Chlorophyll;
- selecting a surface sea height anomaly of 3cm or greater for defining the border of the ocean eddy;
- defining the area enclosed within this border as the initial project location;

b) obtaining baseline measurements, metrics and observations within and beyond the project boundary of:

- sea surface chlorophyll (chl), photosynthetically active radiation (par), sea surface temperature (sst) and sub-surface particulate organic carbon (poc) and dissolved organic carbon (doc) shall be taken.
- sub-surface particulate organic carbon (poc) and dissolved organic carbon (doc) from measurements in the euphotic zone of the sub-surface, and also in proximity of the deep thermocline of the sub-surface;
- calculating the NPP prior to the introduction of iron that shall be considered the background or pre-existing NPP of the initial project boundary;

c) applying an iron compound within the project boundary to enhance photosynthesis within this boundary;

d) obtaining certain measurements, metrics and observations within and adjacent to the project boundary after the introduction of the Iron compound that shall be taken daily or interpolated daily using suite of instruments and sampling mechanisms to create a time-series that defines the area of the actual project boundary on a daily basis from the introduction of iron compound until project conclusion, such as:

- sea surface chlorophyll (chl), photosynthetically active radiation (par), sea surface temperature (sst) and sub-surface particulate organic carbon (poc) and dissolved organic carbon (doc) shall be taken.
- determining the organic carbon flux (corg) as the sum of poc and doc;
- determining the carbon transport efficiency (corgEFF) as corgT/corgE;
- defining CorgEFF(P) as carbon transport efficiency within the project boundary and CorgEFF(B) as the carbon transport efficiency outside the project boundary;
- defining the project duration (Days(P)) as the number of days from introduction of iron compound until project conclusion;

and

e) determining the net quantity of atmospheric carbon that is sequestered by using the measurements obtained from steps b) and d), by calculating the following data:

- the total daily carbon sequestration within the actual project boundary is Cseq(P) = CorgEFF(P) * NPP;
- the total daily carbon sequestration outside the actual project boundary is Cseq(B) = CorgEFF(B) * NPP;
- the total net carbon sequestration is Cseq(NET) = Cseq(P)-Cseq(B);
- the total net carbon sequestration of the project (Ctotal) is the daily Cseq(NET) summed daily, from the introduction of iron compound until project conclusion.

## ILLUSTRATIONS

Example 1:

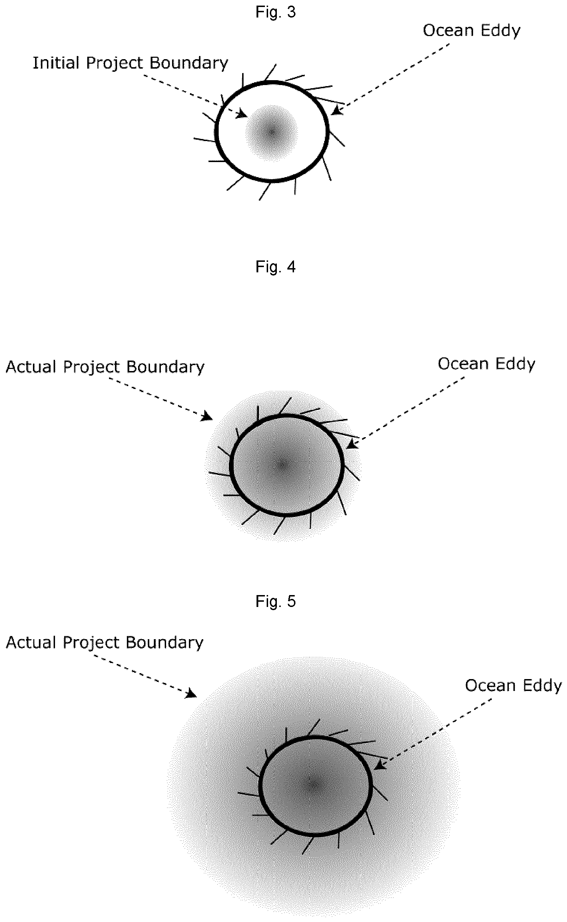

Iron compound is placed within the Ocean Eddy at the onset of the project, resulting in an increase in Net Primary Production. This area, within the Ocean Eddy, is defined as the Initial Project Boundary (Figure 3).

As Net Primary Production increases, the project area will spread beyond the Initial Project Boundary. The actual project boundary will be defined as the delineation between a 10% or greater increase in NPP from its initial value and surrounding waters. As shown in Figure 4, the actual project boundary will change on a day to day basis whereas the Ocean eddy area remains the same or very similar as the beginning of the project.

As the area contained within the Actual Project Boundary increases over time, the Net Primary Production will increase, plateau, and begin to decrease. A point will be reached where the Net Primary Production of the water enclosed within the boundary will cease to be 10% greater that surrounding waters, thus terminating the project (see Figure 5).

## Claims

1. A method for determining net quantity of sequestered atmospheric carbon through ocean iron fertilization comprising the steps of:

a) defining a project boundary;
b) obtaining baseline measurements, metrics and observations within and beyond the project boundary; wherein the baseline measurements, metrics and observations are:

- chlorophyll concentration (chl),
- photosynthetic active radiation (par),
- surface sea temperature (sst),
- day length,
- particulate Organic Carbon (POC),
- dissolved Organic Carbon (DOC),
- euphotic zone Organic carbon (CorgE), and
- Organic carbon close to deep Thermocline (CorgT);

c) applying an iron compound within the project boundary to enhance photosynthesis;
d) obtaining certain measurements, metrics and observations within and adjacent to the project boundary after the introduction of the iron compound to create a time-series that defines the area of the project boundary on a daily basis from introduction of the iron compound until project conclusion;

e) by using the measurements obtained from steps b) and d), determining total daily carbon sequestration within the actual project boundary, Cseq(P), wherein said Cseq(P)= CorgEff (P) * NPP;

f) determining a total daily carbon sequestration outside the project boundary, Cseq(B), wherein said Cseq(B)= CorgEff (B) * NPP;

g) determining total daily net carbon sequestration, Cseq(NET), wherein said Cseq(NET)= Cseq(P)-Cseq(B); and

h) obtaining the total net carbon sequestration of the project boundary (Ctotal) as the sum of the daily Cseq(NET) from the introduction of the iron compound until project conclusion;

and wherein,

- Corg = POC + DOC;
- CorgEff = CorgT/CorgE; and
- NPP is the Net Primary Production defined as:

$$NPP = chl * pb\_opt * day\ length * [0.66125 * \frac{par}{(par + 4.1)}] * z\_eu$$ ;

wherein
- pb_obt is the photosynthetic activity a function of the sea surface temperature (sst):

$$pb\_opt = 1.54 * 10^{[(0.0275 * sst) - 0.07]}$$ ;

and
- z_eu is the euphotic depth at which 1% of the surface/incident light is available, wherein:

where $z\_eu = 200 * (chl\_tot)^{-0.293}$
if chl < 1 , $chl\_tot = 38 * chl^{0.425}$
if chl >= 1, $chl\_tot = 40.2 * chl^{0.507}$
and if z_eu <= 102,

then $z\_eu = 568.2 * (chl\_tot)^{-0.746}$.

2. The method according to claim 1, wherein the project boundary is settled based in the following steps:

a) selecting as a project location an ocean eddy;
b) obtaining a baseline of Net Primary Production (NPP) within the ocean eddy prior to iron introduction;
c) waiting until the iron introduction is performed, where after the NPP reaches a maximum value within the ocean eddy the NPP will decrease until equal to the NPP of adjacent waters; and
d) delimiting the project boundary around the iron enriched ocean eddy, where the NPP is 10% or greater than the adjacent waters.

3. The method according to claim 2, wherein the ocean eddy exists within a region of pelagic ocean that is considered to be High Nutrient Low Chlorophyll.

4. The method according to claim 1, wherein data collection is conducted using an autonomous underwater vehicle (AUV) data collection concurrently with satellite observations, to provide greater resolution of sea metrics.

5. The method according to claim 1, wherein data collection is obtained from public or private data sources, or is obtained from at least one device equipped with instrumentation capable of measuring Cseq(P), Cseq(B) and Cseq(NET); or is obtained from a surface vessel which has been equipped with instrumentation able to measure Cseq(P), Cseq(B) and Cseq(NET).

6. The method according to claim 5, where the at least one device is comprised in the group of: autonomous underwater vehicles (AUV's), sediment traps, buoys, shipboard instruments, niskin bottles and satellite instruments.

7. The method according to claim 1, wherein the observations have a minimum geographical resolution of 10 square kilometers per observation or better.

8. The method according to claim 1, wherein the iron compound is introduced into the project boundary in order to stimulate photosynthetic activity within the project boundary.

9. The method according to claim 2 or claim 3, wherein calculations of NPP within the project boundary are performed using actual, interpolated or extrapolated data and are applied to provide a measurement of the NPP within the project boundary.

**Patentansprüche**

1. Ein Verfahren zur Bestimmung der Nettomenge von sequestriertem atmosphärischem Kohlenstoff durch Ozeaneisendüngung
   das die folgenden Schritte umfasst:

   a) Definieren einer Projektgrenze;
   b) Erfassen von Basislinienmessungen, Metriken und Beobachtungen innerhalb und außerhalb der Projektgrenzen;
   wobei die Basislinienmessungen, Metriken und Beobachtungen folgende sind:

   - Chlorophyll-Konzentration (chi),
   - photosynthetisch aktive Strahlung (par),
   - Meeresoberflächentemperatur (sst),
   - Tageslänge,
   - partikulärer organischer Kohlenstoff (POC),
   - gelöster organischer Kohlenstoff (DOC),
   - Organischer Kohlenstoff der euphotischen Zone (CorgE), und
   - Organischer Kohlenstoff in der Nähe der tiefen Thermokline (CorgT);

   c) Aufbringen einer Eisenverbindung innerhalb der Projektgrenze zur Förderung der Photosynthese;
   d) Erfassen bestimmter Messungen, Metriken und Beobachtungen innerhalb und angrenzend an die Projektgrenze nach dem Einbringen der Eisenverbindung, um eine Zeitreihe zu erstellen, die den Bereich der Projektgrenze auf täglicher Basis vom Einbringen der Eisenverbindung bis zum Projektabschluss definiert;
   e) unter Verwendung der in den Schritten b) und d) erhaltenen Messungen, Bestimmen der gesamten täglichen Kohlenstoffsequestrierung innerhalb der tatsächlichen Projektgrenze, Cseq(P), wobei Cseq(P) = CorgEff (P) * NPP;
   f) Bestimmen einer gesamten täglichen Kohlenstoffsequestrierung außerhalb der Projektgrenze, Cseq(B), wobei Cseq(B) = CorgEff (B) * NPP;
   g) Bestimmen der gesamten täglichen Netto-Kohlenstoffsequestrierung, Cseq(NET), wobei Cseq(NET)= Cseq(P)-Cseq(B); und
   h) Erhalt der gesamten Netto-Kohlenstoffsequestrierung der Projektgrenze (Ctotal) als Summe der täglichen Cseq(NET) von der Einführung der Eisenverbindung bis zum Projektabschluss;
   und wobei,

   - Corg = POC + DOC;
   - CorgEff = CorgT/CorgE; und
   - NPP ist die Nettoprimärproduktion, definiert als:

$$NPP = chl * pb\_opt * Tageslänge * \left[0{,}66125 * \frac{par}{(par+4{,}1)}\right] * z\_eu$$

   wobei

**14**

- pb_obt ist die photosynthetische Aktivität eine Funktion der Meeresoberflächentemperatur (sst):

$$pb\_opt = 1,54 * 10^{|(0,0275\,*\,sst)-0,07|} \text{ ;}$$

und
- z_eu ist die euphotische Tiefe, in der 1 % des Oberflächen-/Einfallslichts verfügbar ist, wobei:

wobei $z\_eu = 200 * (chl\_tot)^{-0,293}$
wenn $chl < 1$ , $chl\_tot = 38 * chl^{0.425}$
wenn $chl >= 1$, $chl\_tot = 40,2 * chl^{0.507}$
und wenn $z\_eu <= 102$,

dann ist $z\_eu = 568,2 * (chl\_tot)^{-0,746}$.

2. Das Verfahren nach Anspruch 1, wobei die Projektgrenze in den folgenden Schritten festgelegt wird:

a) Auswahl eines Ozeanwirbels als Projektstandort;
b) Erhalten einer Basislinie der Nettoprimärproduktion (NPP) innerhalb des Ozeanwirbels vor der Eiseneinbringung;
c) Abwarten, bis die Eiseneinbringung erfolgt ist, wobei nach Erreichen eines Maximalwertes der NPP innerhalb des Ozeanwirbels die NPP abnehmen wird, bis sie der NPP der angrenzenden Gewässer entspricht; und
d) Abgrenzung der Projektgrenze um den eisenangereicherten Ozeanwirbel, in dem die NPP 10 % oder größer ist als in den angrenzenden Gewässern.

3. Das Verfahren nach Anspruch 2, wobei der Ozeanwirbel innerhalb einer Region des pelagischen Ozeans liegt, die als nährstoffreich und chlorophyllarm gilt.

4. Das Verfahren nach Anspruch 1, wobei die Datenerfassung unter Verwendung eines autonomen Unterwasserfahrzeugs (AUV) gleichzeitig mit Satellitenbeobachtungen durchgeführt wird, um eine höhere Auflösung der Meeresmetriken zu erhalten.

5. Das Verfahren nach Anspruch 1, wobei die Datenerfassung von öffentlichen oder privaten Datenquellen erhalten wird oder von mindestens einem Gerät erhalten wird, das mit einer Instrumentierung ausgestattet ist, die in der Lage ist, Cseq(P), Cseq(B) und Cseq(NET) zu messen; oder von einem Oberflächenschiff erhalten wird, das mit einer Instrumentierung ausgestattet wurde, die in der Lage ist, Cseq(P), Cseq(B) und Cseq(NET) zu messen.

6. Das Verfahren nach Anspruch 5, wobei das mindestens eine Gerät aus der Gruppe der autonomen Unterwasserfahrzeuge (AUVs), Sedimentfallen, Bojen, Schiffsinstrumente, Niskin-Flaschen und Satelliteninstrumente stammt.

7. Verfahren nach Anspruch 1, wobei die Beobachtungen eine minimale geographische Auflösung von 10 Quadratkilometern pro Beobachtung oder besser haben.

8. Das Verfahren nach Anspruch 1, wobei die Eisenverbindung in die Projektgrenze eingebracht wird, um die photosynthetische Aktivität innerhalb der Projektgrenze zu stimulieren.

9. Das Verfahren nach Anspruch 2 oder Anspruch 3, wobei die Berechnungen der NPP innerhalb der Projektgrenze unter Verwendung von tatsächlichen, interpolierten oder extrapolierten Daten durchgeführt werden und angewendet werden, um eine Messung der NPP innerhalb der Projektgrenze bereitzustellen.

**Revendications**

1. Procédé pour déterminer la quantité nette de carbone atmosphérique séquestré par la fertilisation ferreuse de l'océan, comprenant les étapes de :

a) la définition d'une limite de projet ;
b) l'obtention des mesures, des paramètres et des observations de base à l'intérieur et au-delà de la limite du projet ;
dans lequel les mesures, les paramètres et les observations de base sont :

- concentration de chlorophylle (chi),
- rayonnement actif photosynthétique (PAR),
- température de surface de la mer (sst),
- durée du jour,
- carbone organique particulaire (COP),
- carbone organique dissous (COD),
- carbone organique de la zone euphotique (CorgE), et
- Carbone organique près de la thermocline profonde (CorgT) ;

c) l'application d'un composé de fer à l'intérieur de la limite du projet pour améliorer la photosynthèse ;
d) l'obtention de certaines mesures, métriques et observations à l'intérieur et à proximité de la limite du projet après l'introduction du composé de fer pour créer une série chronologique qui définit la zone de la limite du projet sur une base quotidienne depuis l'introduction du composé de fer jusqu'à la conclusion du projet ;
e) la détermination de la séquestration quotidienne totale du carbone à l'intérieur de la limite réelle du projet, Cseq(P), où ledit Cseq(P)= CorgEff (P) * PPN , en utilisant les mesures obtenues à partir des étapes b) et d);
f) la détermination d'une séquestration quotidienne totale du carbone à l'extérieur de la limite du projet, Cseq(B), où ladite Cseq(B)= CorgEff (B) * PPN ;
g) la détermination de la séquestration quotidienne totale du carbone net, Cseq(NET), où ladite Cseq(NET)= Cseq(P)-Cseq(B) ; et
h) l'obtention de la séquestration nette totale du carbone de la limite du projet (Ctotal) comme la somme des Cseq(NET) quotidiens depuis l'introduction du composé de fer jusqu'à la conclusion du projet ;
et dans lequel,

- Corg = COP + COD ;
- CorgEff = CorgT/CorgE; et
- la PPN est la production primaire nette définie comme:

$$PPN = chl * pb\_opt * longueur\ du\ jour * [0.66125 * \frac{par}{(par+4.1)}] * z\_eu$$

dans lequel :

- pb_obt est l'activité photosynthétique en fonction de la température de surface de la mer (sst) :

$$pb\_opt = 1.54 * 10^{|(0.0275 * sst)-0.07|} ;$$

et
- z_eu est la profondeur euphotique à laquelle 1% de la lumière de surface/incidente est disponible, où :

où $z\_eu = 200 * (chl\_tot)^{-0,293}$
si $chl < 1$ , $chl\_tot = 38 * chl^{0.425}$
si $chl >= 1$, $chl\_tot = 40.2 * chl^{0.507}$
et si $z\_eu <= 102$,

alors $z\_eu = 568.2 * (chl\_tot)^{-0.746}$.

2. Procédé selon la revendication 1, dans lequel la limite du projet est établie sur la base des étapes suivantes :

a) la sélection d'un tourbillon océanique comme emplacement du projet ;
b) l'obtention d'une ligne de base de la production primaire nette (PPN) dans le tourbillon océanique avant l'introduction du fer ;
c) l'attente jusqu'à ce que l'introduction du fer soit effectuée, où après que la PPN ait atteint une valeur maximale dans le tourbillon océanique, la PPN diminuera jusqu'à être égale à la PPN des eaux adjacentes ; et

d) la délimitation de la limite du projet autour du tourbillon océanique enrichi en fer, où la PPN est de 10% ou plus que les eaux adjacentes.

3.  Procédé selon la revendication 2, dans lequel le tourbillon océanique existe dans une région de l'océan pélagique qui est considérée comme étant à haute teneur en nutriments et à faible teneur en chlorophylle.

4.  Procédé selon la revendication 1, dans lequel la collecte de données est effectuée à l'aide d'un véhicule sous-marin autonome (AUV) en même temps que les observations par satellite, pour fournir une plus grande résolution des métriques de la mer.

5.  Procédé selon la revendication 1, dans lequel la collecte de données est obtenue à partir de sources de données publiques ou privées, ou est obtenue à partir d'au moins un dispositif équipé d'une instrumentation capable de mesurer Cseq(P), Cseq(B) et Cseq(NET) ; ou est obtenue à partir d'un navire de surface qui a été équipé d'une instrumentation capable de mesurer Cseq(P), Cseq(B) et Cseq(NET).

6.  Procédé selon la revendication 5, où le au moins un dispositif est compris dans le groupe de : véhicules sous-marins autonomes (AUV), pièges à sédiments, bouées, instruments de bord, bouteilles de Niskin et instruments satellites.

7.  Procédé selon la revendication 1, dans lequel les observations ont une résolution géographique minimale de 10 kilomètres carrés par observation ou mieux.

8.  Procédé selon la revendication 1, dans lequel le composé de fer est introduit dans la limite du projet afin de stimuler l'activité photosynthétique dans la limite du projet.

9.  Procédé selon la revendication 2 ou la revendication 3, dans lequel les calculs de la PPN à l'intérieur de la limite du projet sont effectués en utilisant des données réelles, interpolées ou extrapolées et sont appliqués pour fournir une mesure de la PPN à l'intérieur de la limite du projet.

## Fig. 1A

## Fig. 1B

Sea Surface Height Anomaly (cm)

Fig. 2

Fig. 3

Initial Project Boundary

Ocean Eddy

Fig. 4

Actual Project Boundary

Ocean Eddy

Fig. 5

Actual Project Boundary

Ocean Eddy

## REFERENCES CITED IN THE DESCRIPTION

**Non-patent literature cited in the description**

- **SMETACEK, V. et al.** Deep carbon export from a Southern Ocean iron- fertilized diatom bloom. *Nature,* 2012, vol. 487, 313-319
- **SABINE, C.L. et al.** The oceanic sink for anthropogenic CO2. *Science,* 2004, vol. 305, 367-371
- **BOYD, P.W. et al.** The decline and fate of an iron-induced subarctic phytoplankton bloom. *Nature,* 2004, vol. 428, 549-553
- **BISHOP, J. et al.** Robotic observations of enhanced carbon biomass and export at 55°S during SOFeX. *Science,* 2004, vol. 304, 417-420
- **WILLIAMSON, P. et al.** Ocean Fertilization for geoengineering: A review of effectiveness, environmental impacts and emerging governance. *Process Safety and Environmental Protection,* 2012, 475-488
- **BLAIN, S. et al.** Effect of natural iron fertilization on carbon sequestration in the Southern Ocean. *Nature,* 26 April 2007, vol. 446, 1070-1074
- **BUESSELER, K.O. et al.** Revisiting carbon flux through the Ocean's twilight zone. *Science,* 2007, vol. 316 (5824), 567-570
- **JIN, X. et al.** The impact on atmospheric CO2 of iron fertilization induced changes in the ocean's biological pump. *Biogeosciences,* 2008, vol. 5, 385-406
- **AUMONT, O. ; L. BOPP.** Globalizing results from ocean in situ iron fertilization studies. Global Biogeochem. *Cycles,* 2006, 20
- **ZAHARIEV, K. ; J.R. CHRISTIAN ; K.L. DENMAN.** Preindustrial, historical, and fertilization simulations using a global ocean carbon model with new parameterizations of iron limitation, calcification, and N2 fixation. *Progress in Oceanography,* 2008, vol. 77, 56-82

- **ENGLAND, M.H.** The Age of Water and Ventilation Timescales in a Global Ocean Model. *Journal of Physical Oceanography,* 25 November 1995, 2756-2777
- IPCC, Climate Change 1994: Radiative Forcing of Climate Change and an Evaluation of the IPCC IS92 Emission Scenarios. Cambridge University Press, 1995
- **MATSUMOTO, K.** Radiocarbon based circulation age of the world oceans. *Journal of Geophysical Research - Oceans,* 2007, vol. 112 (C09004
- UNFCCC, REPORT OF THE CONFERENCE OF THE PARTIES ON ITS THIRD SESSION. 01 December 1997
- **FINE R.** Observations of CFCs and SF6 as Ocean Tracers. *Annual Review in Marine Science,* 2011, vol. 3, 173-195
- **KHATIWALA, S. ; F. PRIMEAU ; M. HOLZER.** Ventilation of the deep ocean constrained with tracer observations and implications for radiocarbon estimates of ideal mean age. *Earth and Planetary Science Letters,* 2012, vol. 325-326, 116-125
- **EPPLEY, RW.** Temperature and phytoplankton growth in the sea. *Fishery Bulletin,* 1972, vol. 70, 1063-1085
- **BEHRENFELD, MJ. ; PG FALKOWSKI.** Photosynthetic rates derived from satellite-based chlorophyll concentration. *Limnology and Oceanography,* 1997, vol. 42, 1-20
- **MOREL, A. ; J-F BERTHON.** Surface pigments, algal biomass profiles, and potential production of the euphotic layer: Relationships reinvestigated in view of remote-sensing applications. *Limnology and Oceanography,* 1989, vol. 34, 1545-1562